(19) Europäisches Patentamt
European Patent Office
Office européen des brevets

(11) Veröffentlichungsnummer: **0 043 507**
**B1**

(12) # EUROPÄISCHE PATENTSCHRIFT

(45) Veröffentlichungstag der Patentschrift: **22.08.84**

(51) Int. Cl.³: **C 07 D 319/08**, C 11 B 9/00

(21) Anmeldenummer: **81104914.7**

(22) Anmeldetag: **25.06.81**

(54) **2,4-Dioxa-7,10-methano-spiro(5,5)undecane, deren Herstellung sowie diese enthaltende Riechstoffkompositionen.**

(30) Priorität: **03.07.80 DE 3025187**

(43) Veröffentlichungstag der Anmeldung: **13.01.82 Patentblatt 82/2**

(45) Bekanntmachung des Hinweises auf die Patenterteilung: **22.08.84 Patentblatt 84/34**

(84) Benannte Vertragsstaaten: **CH DE FR GB LI NL**

(56) Entgegenhaltungen:
**DE - A - 2 604 553**
**GB - A - 917 027**
**NL - C - 126 595**

**CHEMICAL ABSTRACTS, Band 92, Chemical Substance Index, Pp-Z, Januar-Juni 1980, 5420CS "Spiro (bicyclo(2.2.1)heptane-2,5'-(1,3)dioxane" Z.Chem. 1979, 19(10), 373**
**Chem.Abstr. Ring Index Supplement III (1962-1963) S.131, 12361**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Bruns, Klaus, Dr., Notburgaweg 6, D-4150 Krefeld-Traar (DE)**
Erfinder: **Dang, Thuy Nguyen, Elisabethstrasse 140, D-4150 Krefeld-Traar (DE)**

**EP 0 043 507 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Es wurde gefunden, dass Isomergemische von 2,4-Dioxa-7,10-methano-spiro[5.5]undecanen der Formel

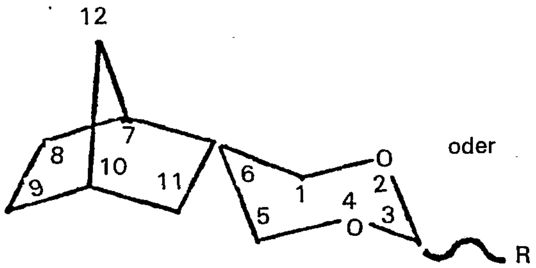

oder

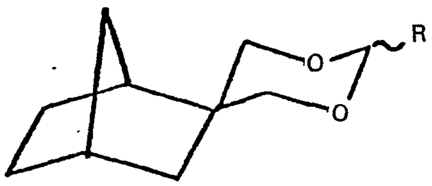

worin

R = Wasserstoff, Alkyl $C_1$-$C_4$, Vinyl oder Propenyl,

~ = equatorial oder axial ist,

ausgezeichnete neue Riechstoffe zur Herstellung wertvoller Kompositionen für die Parfümierung kosmetischer Präparate und Reinigungsmittel sind.

Die Herstellung der erfindungsgemässen neuen Verbindungen erfolgt nach bekannten Verfahren der organischen Synthese in mehreren Stufen, wobei im ersten Schritt Cyclopentadien mit Acrolein in den Norborn-5-en-2-carbaldehyd überführt, dieser im zweiten Schritt durch Methylolierung nach Canizzaro im alkalischen Medium zum 2,2-di-Hydroxymethyl-norborn-5-en umgesetzt, in diesem in dritter Stufe die Doppelbindung katalytisch hydriert und in nachfolgender Acetalisierung mit entsprechenden Aldehyden in vierter Stufe das erfindungsgemässe Spiroacetal erhalten wird.

Die Reaktion verläuft dabei nach folgendem Schema, wobei R und ~ die vorgenannte Bedeutung besitzen:

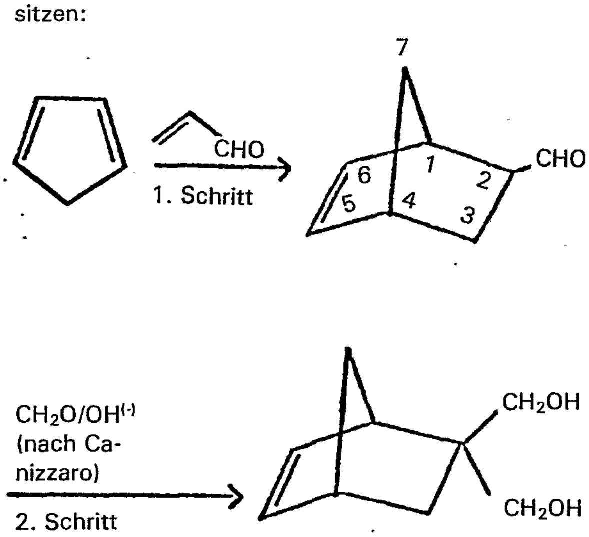


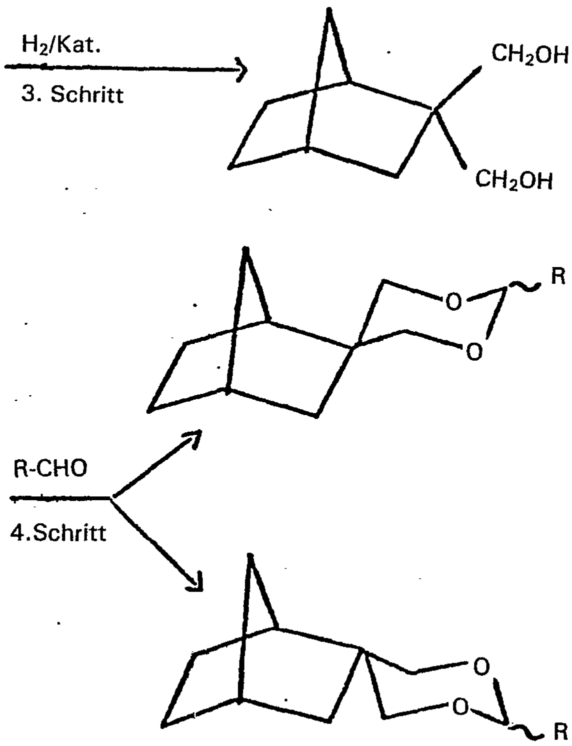


Die erfindungsgemässen neuen 2,4-Dioxa-7,10-methano-spiro[5.5]undecane werden dabei als Gemisch stereoisomerer Formen erhalten und als solche als Riechstoffe verwendet und als Komponenten in Riechstoffkompositionen eingesetzt.

Als erfindungsgemässe neue Riechstoffe sind zum Beispiel 2,4-Dioxa-7,10-methano-, 2,4-Dioxa-3-methyl-7,10-methano-, 2,4-Dioxa-3-ethyl-7,10-methano-, 2,4-Dioxa-3-isopropyl-7,10-methano-, 2,4-Dioxa-3-vinyl-7,10-methano-, 2,4-Dioxa-3-propenyl-7,10-methano-spiro[5.5]undecan zu nennen.

Die erfindungsgemässen neuen Riechstoffe zeichnen sich durch intensive, interessante unterschiedliche Geruchsnoten von hoher Geruchsqualität und Geruchsfülle aus. Ein weiterer Vorteil der neuen Riechstoffe ist ihre sehr gute Kombinationsfähigkeit zu neuartigen Geruchsnuancen.

Die erfindungsgemässen neuen Riechstoffe können mit anderen Riechstoffen in verschiedensten Mengenverhältnissen zu neuen Riechstoffkompositionen gemischt werden. Im allgemeinen wird sich jedoch der Anteil der neuen Riechstoffe in den Riechstoffkompositionen in den Mengen von 1 bis 50 Gewichtsprozent, bezogen auf die gesamte Komposition, bewegen. Derartige Kompositionen können direkt als Parfüm oder auch zur Parfümierung von Kosmetika wie Cremes, Lotionen, Duftwässern, Aerosolen und Toiletteseifen dienen. Sie können aber auch zur Geruchsverbesserung technischer Produkte wie Wasch- und Reinigungsmittel, Desinfektionsmittel und Textilbehandlungsmittel eingesetzt werden.

Aus Chem. Abstracts 92 : 75936 t (1980) sind Spiroacetale bekannt (2,4-Dioxa-7,10-methano-spiro-[5.5]undec-8-ene), die aufgrund der Doppelbindung in 8,9-Position keine interessanten Riechstoffqualitäten aufweisen. Ferner sind in DE-A-2 604 553 2,4-Dioxa-spiro-[5.5]undec-8-ene beschrieben, die jedoch infolge ihrer andersartigen Struktur (Fehlen

der Methylenbrücke, Doppelbindung in 8,9-Position) gegenüber den beanspruchten Verbindungen veränderte bzw. deutlich unterschiedliche Geruchscharakteristika aufweisen.

Die nachfolgenden Beispiele sollen den Gegenstand der Erfindung näher erläutern.

Zunächst wird die Herstellung der neuen Riechstoffe beschrieben.

*Beispiel 1*

*2,4-Dioxa-3-methyl-7,10-methano-spiro[5.5]-undecan*

*1.Schritt:* Herstellung von Norborn-5-en-2-carbaldehyd.

Zu 33,05 g frisch destilliertem Cyclopentadien, gewonnen durch Destillation von Bicyclopentadien über eine 50 cm Vigreux-Kolonne bei Normaldruck, wurden 28,03 g Acrolein während 30 Minuten unter Rühren zugetropft. Sobald die Temperatur 55°C übersteigt, wurde das Reaktionsgefäss mit Eiswasser gekühlt. Nach beendeter Zugabe wurde noch 3 Stunden lang bei 60°C nachgerührt und anschliessend im Wasserstrahlvakuum destilliert. Das Zwischenprodukt wurde mit einer Ausbeute von 73,8% der Theorie erhalten und besass folgende Kennzahlen:

$Kp_{17}$ 61 °C; GC: 89,3/10,6%.
IR (film): 2710, 1720/cm (CHO): 3060, 720/cm (cis-CH = CH-)
$^1$H-NMR ($CDCl_3$): 9,47 ppm (d, 1H) (CHO); 6,18 ppm (doppeltes d, 2H) ($\underline{H}C=C\underline{H}$).

*2.Schritt:* Herstellung von 2,2-di-Hydroxymethyl--norborn-5-en.

Zu einer Mischung aus 42 ml 37%igem Formalin und 12,3 g Natriumhydroxid, gelöst in 36,9 ml Wasser, werden unter Rühren in Stickstoffatmosphäre und Wasserkühlung während 20 Minuten 25 g Norborn-5-en-2-carbaldehyd zugetropft. Die Temperatur des Reaktionsgemisches stieg dabei auf 30°C. Anschliessend wurde eine Stunde bei 55°C gerührt und danach das Reaktionsgemisch mit 50 ml Methylisobutylketon extrahiert, die organische Phase mit 50 ml Wasser und die wässrige Phase mit 50 ml Methylisobutylketon gewaschen. Die vereinigten organischen Phasen wurden neutral gewaschen, über Natriumsulfat getrocknet und das Lösungsmittel abdestilliert. Der Rückstand wurde aus Petrolether (60 bis 95)/Toluol (1:1) umkristallisiert. Das Zwischenprodukt wurde in Gestalt farbloser Kristalle mit einer Ausbeute von 77,6% der Theorie erhalten und besass folgende Kennzahlen:

Fp 113,5-115°C
IR (KBr): 3300/cm (OH); 1037, 1018/cm (prim. OH): 3060, 715 cm (cis-HC = CH-)
$^1$H-NMR ($d^6$-DMSO): 6,16 ppm (m, 2H) ($\underline{H}C=C\underline{H}$); 4,40 ppm (2t, 2H) ($O\underline{H}$); 3,40 ppm (m, 4H) ($C\underline{H}_2OH$): 0,59 ppm (2d, 1H) ($C_7$-$\underline{H}$).

*3.Schritt:* Herstellung von 2,2-di-Hydroxymethyl--norboran.

155 g 2,2-di-Hydroxymethyl-norborn-5-en (1,0 Mol) wurden in 400 ml Methanol gelöst, mit 14,2 g Raney-Nickel versetzt und bei 45°C und 70 bar Wasserstoff-Druck 5 Stunden lang bis zur Sättigung im Autoklaven hydriert. Nach Entfernen von Katalysator und Lösungsmittel hinterblieb das 2,2-di-Hydroxymethyl-norboran in quantitativer Ausbeute in Gestalt farbloser Kristalle mit folgenden Kennzahlen:

Fp 97-98°C
IR (KBr) 3330/cm (OH), 1020/cm (prim. OH)
$^1$H-NMR ($CDCl_3$): 3,65 ppm (2m, 5H) ($C\underline{H}_2O\underline{H}$): 0,72 ppm (2d, 1H) ($C_7$-$\underline{H}$).

*4.Schritt:* Herstellung von 2,4-Dioxa-3-methyl--7,10-methano-spiro[5.5]undecan.

Eine Mischung aus 90,8 g 2,2-di-Hydroxymethyl-norboran, 86,1 g Orthoameisensäuretriethylester und 25,6 g Acetaldehyd (Molverhältnis 1:1:1) wurde mit 0,1 g p-Toluolsulfonsäure versetzt, wobei leichte Erwärmung eintrat. Nach Rühren über Nacht bei Raumtemperatur wurden Ameisensäureethylester und Ethanol abdestilliert. Der Rückstand wurde in Ether aufgenommen, mit 2n-Sodalösung und Wasser gewaschen und über Natriumsulfat getrocknet. Nach Abdestillieren des Lösungsmittels wurde der Rückstand im Wasserstrahlvakuum destilliert. Das mit 90%iger Ausbeute als farblose Flüssigkeit erhaltene 2,4-Dioxa-3-methyl-7,10-methano-spiro[5.5]-undecan wies folgende Kenndaten auf:

$Kp_{17}$ 110°C; GC 78,4/21,1%; $n_D^{20}$ 1,4822
IR (Film): 1150, 1100, 1025/cm (-O-C-O-); 1405/cm ($CH_3$)
$^1$H-NMR ($CDCl_3$): 4,60 ppm (q, 1H) ($C_3$-$\underline{H}$), 3,68 ppm (2m, 4H) [$C\underline{H}_2$-O-CH($CH_3$)-O-$C\underline{H}_2$]; 0,63 ppm (2d, 1H) ($C_{12}$-H).

Das Produkt, welches ein Gemisch zweier Stereoisomerer darstellt, besitzt eine Rhabarber-, Galbanum-Geruchsnote.

*Beispiel 2*

*2,4-Dioxa-7,10-methano-spiro[5.5]undecan*

Die Herstellung erfolgte völlig analog den Angaben in Beispiel 1, wobei jedoch im 4.Schritt anstelle des Acetaldehyds mit der entsprechenden Menge Paraformaldehyd in Ethylenchlorid direkt acetalisiert wurde. Das als farblose Flüssigkeit erhaltene Endprodukt besass folgende Kenndaten:

$KP_{17}$ 104°C, $n_D^{20}$ 1,4932, MG: 168 (MS einheitlich)
Geruch: minzig, Menthon-Note.

*Beispiel 3*

*2,4-Dioxa-3-ethyl-7,10-methano-spiro[5.5]-undecan*

Die Herstellung erfolgte analog den Angaben in Beispiel 1, wobei im 4.Schritt anstelle des Acetaldehyds die entsprechende Menge Propionaldehyd eingesetzt wurde. Das als farblose Flüssigkeit erhaltene Endprodukt wies folgende Kenndaten auf:

$Kp_{17}$ 124°C,$n_D^{20}$1,4813, MG 196 (GC/MS: 2 Stereoisomere)
Geruch: holzig, blumig, süss, Kunsthonig-, Boisambrene-Note.

*Beispiel 4*

*2,4-Dioxa-3-i-propyl-7,10-methano-spiro[5.5]-undecan*

Die Herstellung erfolgte analog den Angaben in

Beispiel 1, wobei jedoch im 4.Schritt anstelle des Acetaldehyds die entsprechende Menge Isobutyraldehyd verwendet wurde. Das erhaltene Endprodukt stellt eine farblose Flüssigkeit mit folgenden Kenndaten dar:
$KP_{17}$ 128 °C, $n_D^{20}$ 1,4783, MG: 210 (GC/MS: 2 Stereoisomere)
Geruch: erdig, holzig, süss.

*Beispiel 5*

*2,4-Dioxa-3-vinyl-7,10-methano-spiro[5.5]-undecan*

Die Herstellung erfolgte analog den Angagen in Beispiel 1, wobei im 4.Schritt anstelle des Acetaldehyds die entsprechende Menge Acrolein eingesetzt wurde. Das erhaltene Endprodukt ist eine farblose Flüssigkeit mit folgenden Kenndaten:
$KP_1$ 84 °C, $n_D^{20}$ 1,4955, MG: 194 (GC/MS: 2 Stereoisomere)
Geruch: blumig, Honig-, Diphenylether-Note.

*Beispiel 6*

*2,4-Dioxa-3-(1-propen-1-yl)-7,10-methano-spiro-[5.5]undecan*

Die Herstellung erfolgte analog den Angaben in Beispiel 1, wobei im 4.Schritt anstelle des Acetaldehyds die entsprechende Menge Crotonaldehyd eingesetzt wurde. Das erhaltene Endprodukt stellt eine farblose Flüssigkeit mit folgenden Kennzahlen dar:
$Kp_{0,8}$ 93 °C, $n_D^{20}$ 1,4956, MG: 208 (GC/MS: 4 Stereoisomere)
Geruch: ölig, süss, Sandelholz-Note.

Die in den vorstehenden Beispielen genannten Verbindungen sind für die Herstellung der verschiedensten Riechstoffkompositionen geeignet, die zur Parfümierung der unterschiedlichsten Produkte wie Kosmetika, Waschmittel, Seifen, aber auch technischen Produkten in Konzentrationen von 0,05 bis 2 Gewichtsprozent eingesetzt werden. Nachfolgend werden noch Beispiele für Riechstoffkompositionen mit einem Gehalt an erfindungsgemässen Verbindungen aufgeführt.

*Beispiel 7*

*Hyacinthe-Komposition*

| | | |
|---|---|---|
| 2,4-Dioxa-3-methyl-7,10-methano-spiro-[5.5]undecan | 150 | Gewichtsteile |
| Benzylacetat | 150 | « |
| Linalool | 150 | « |
| Zimtalkohol | 100 | « |
| Phenylethylalkohol | 90 | « |
| Hydroxycitronellal | 90 | « |
| Heliotropin | 50 | « |
| Benzylalkohol | 40 | « |
| Phenylacetaldehyd | 30 | « |
| Ylang-Ylang-Öl | 25 | « |
| Dimethylbenzylcarbinylacetat | 20 | « |
| Eugenol | 15 | « |
| Cinnamylacetat | 15 | « |
| Isoeugenol | 10 | « |
| α-Iron | 10 | « |
| Phenylacetaldehyd-dimethylacetal | 10 | « |
| Citronellol | 10 | « |
| trans-2-Hexanal (10% i.DEP) | 10 | « |
| Hexenylbenzoat | 5 | « |
| Rosenoxid | 5 | « |
| Cyclamenaldehyd | 5 | « |
| Methyloctincarbonat (10% i.DEP) | 5 | « |
| N-Methyl-methylanthranilat | 5 | « |
| | 1000 | Gewichtsteile |

*Beispiel 8*

*Honig-Blumen-Variante*

| | | |
|---|---|---|
| 2,4-Dioxa-3-vinyl-7,10-methano-spiro[5.5]undecan | 100 | Gewichtsteile |
| Phenylethylalkohol | 350 | « |
| Phenylethylphenylacetat | 150 | « |
| Zimtalkohol | 100 | « |
| Hydroxycitronellal | 60 | « |
| Heliotropin | 50 | « |
| Propylphenylacetat | 50 | « |
| Phenylethylsalicylat | 35 | « |
| Linalool | 30 | « |
| Methylphenylacetat | 30 | « |
| p-Methylchinolin | 20 | « |
| Methyleugenol | 5 | « |
| Vanillin | 5 | « |
| Cumarin | 5 | « |
| Ethylpelargonat | 5 | « |
| Citronellol | 5 | « |
| | 1000 | Gewichtsteile |

**Patentansprüche**

1. Spiroacetale der allgemeinen Formel

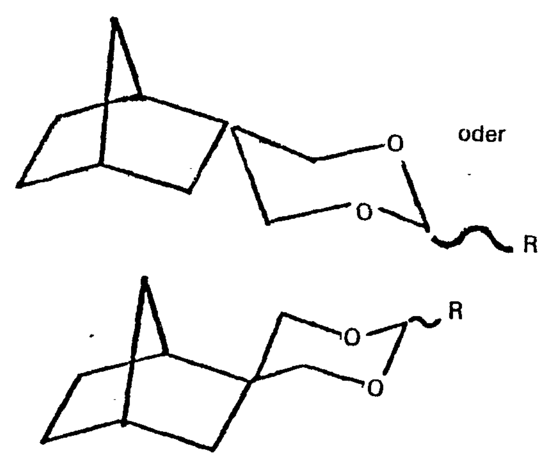

oder

worin
R = Wasserstoff, Alkyl $C_1$-$C_4$, Vinyl oder Propenyl,
~ = equatorial oder axial ist,
in Form der Isomerengemische.

2. Verfahren zur Herstellung der Isomerengemische nach Anspruch 1, dadurch gekennzeichnet, dass die Reaktion nach folgendem Schema verläuft:

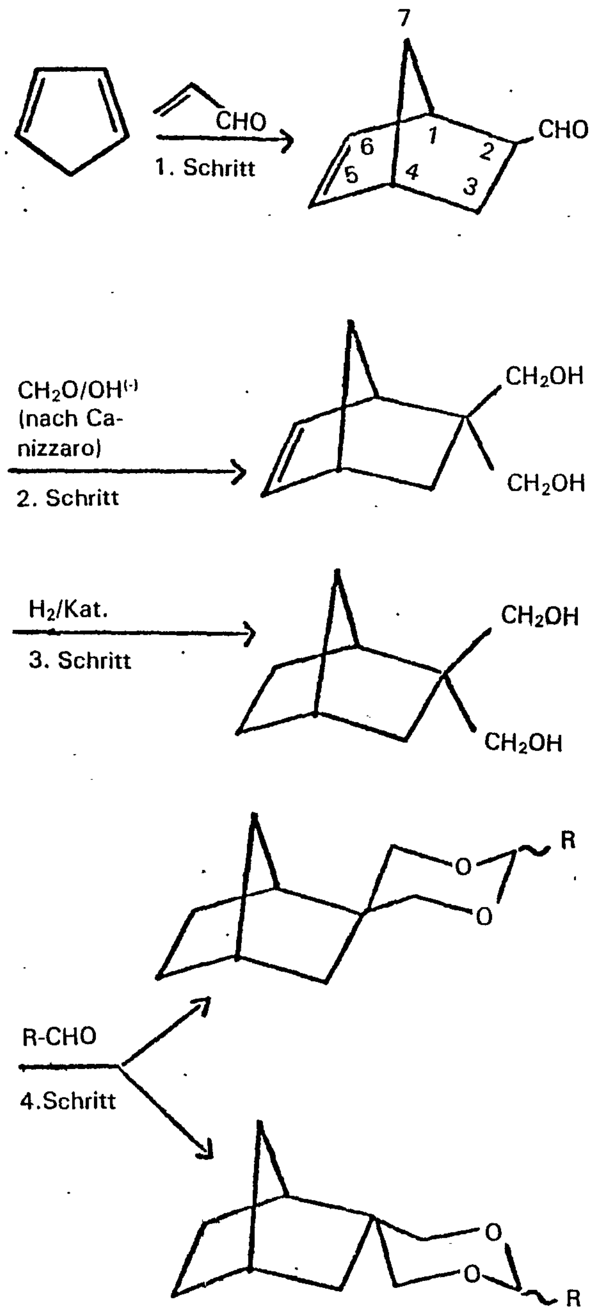


wobei R und ~ die vorgenannte Bedeutung besitzen.

3. Verwendung der Spiroacetale nach Anspruch 1 als Riechstoffe.

4. Riechstoffkompositionen, gekennzeichnet durch einen Gehalt an Spiroacetalen nach Anspruch 1.

5. Riechstoffkompositionen nach Anspruch 4, dadurch gekennzeichnet, dass sie die Spiroacetale in einer Menge von 1 bis 50 Gewichtsprozent, bezogen auf die gesamte Komposition, enthalten.

**Claims**

1. Spiroacetals corresponding to the following general formula

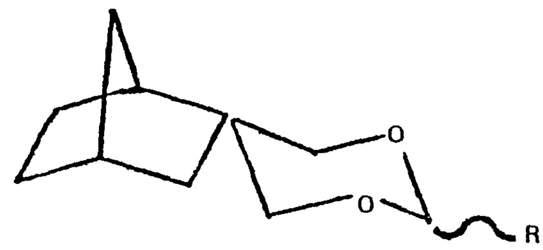

or

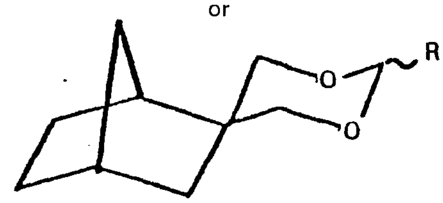

in wich

R represents hydrogen, $C_1$-$C_4$-alkyl, vinyl or propenyl and

~ is equatorial or axial,

in the form of the isomer mixtures.

2. A process for producing the isomer mixtures claimed in claim 1, characterized in that the reaction takes place in accordance with the following scheme

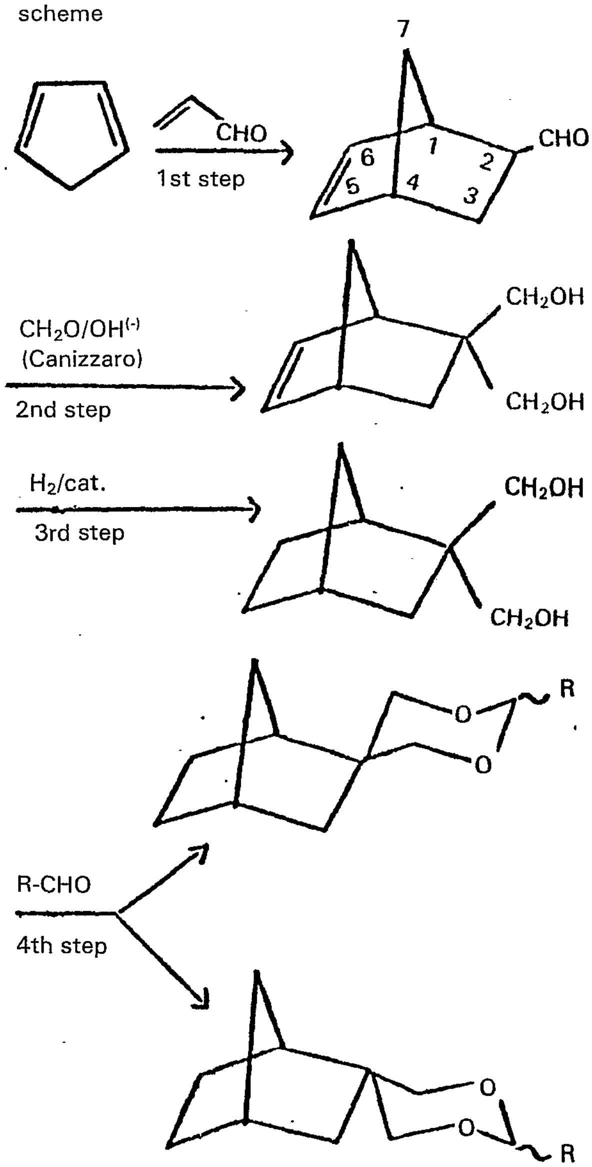

R and ~ having the meanings defined above.

3. The use of the spiroacetals claimed in claim 1 as perfumes.

4. Perfume compositions characterized by a content of the spiroacetals claimed in claim 1.

5. Perfume compositions as claimed in claim 4, characterized in that they contain the spiroacetals in a quantity of from 1 to 50% by weight, based on the composition as a whole.

**Revendications**

1. Spiroacétals de formule générale

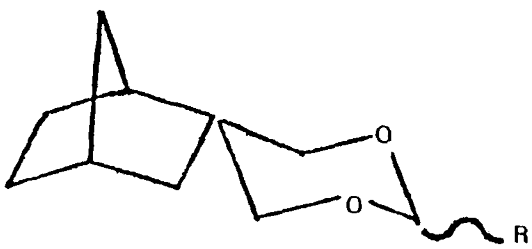

ou

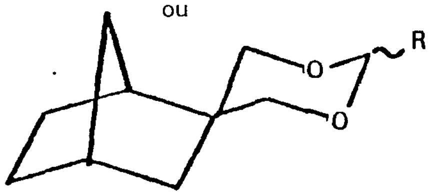

dans laquelle

R = hydrogène, alkyle en $C_1$-$C_4$, vinyle ou propényle,

~ = équatorial ou axial,

à l'état de mélanges d'isomeres.

2. Procédé de préparation des mélanges d'isomères selon la revendication 1, caractérisé en ce que la réaction se déroule selon le schéma suivant:

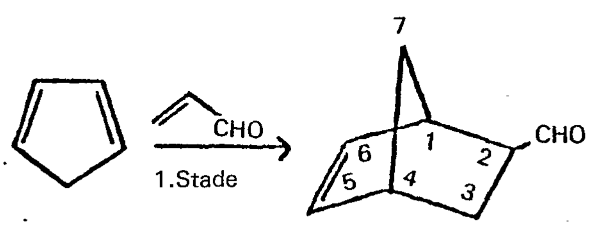


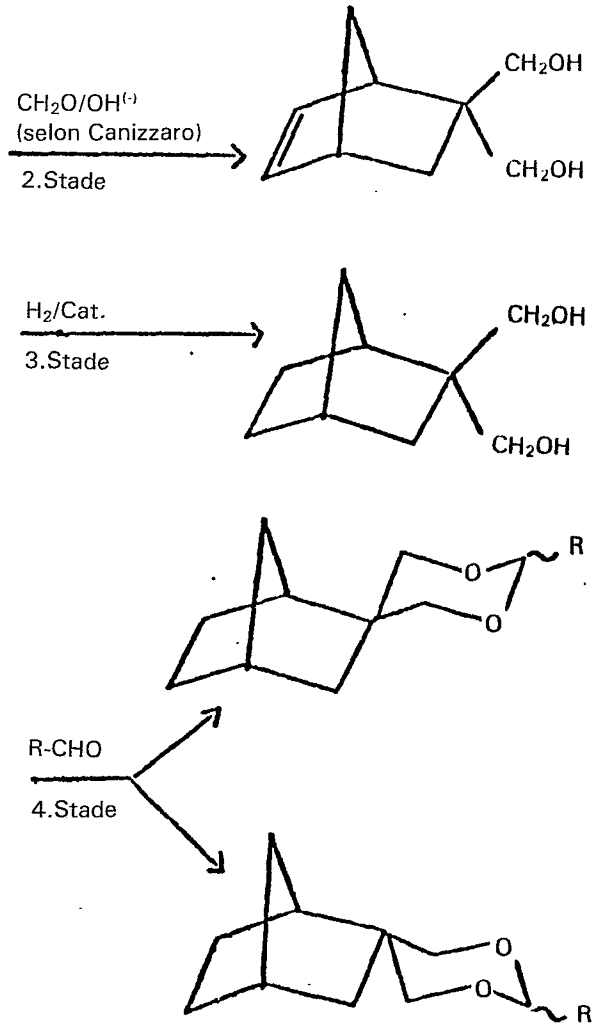


R et ~ ayant les significations indiquées ci-dessus.

3. Utilisation des spiroacétals selon la revendication 1, en tant que matières aromatiques.

4. Compositions de parfums caractérisées en ce qu'elles contiennent des spiroacétals selon la revendication 1.

5. Compositions de parfums selon la revendication 4, caractérisées en ce qu'elles contiennent les spiroacétals en quantités de 1 à 50% du poids de la composition totale.